# EUROPEAN PATENT APPLICATION

(11) **EP 4 435 404 A1**
(43) Date of publication of application: **25.09.2024**
(21) Application number: 21923475.4
(22) Date of filing: 17.11.2021
(51) Int. Cl.: G01N 1/10, C12M 1/28, B01D 35/02

(54) **SAMPLER FOR A BIOREACTOR**

(71) Applicant: Institute for Poliomyelitis, Chumakov Federal Scientific Center of Research and Development of Immune-and-Biological Products of Russian, Moscow, 108819 (RU)
(72) Inventor: IVIN, Yury Yurievich, g. Troitsk, 457103 (RU); PINIAEVA, Anastasia Nikolaevna, Moscow, 108819 (RU); KOVPAK, Anastasia Aleksandrovna, Moscow, 108806 (RU); ISHMUKHAMETOV, Aydar Ayratovich, Moscow, 127473 (RU)
(74) Representative: Koudine, Andreï
(86) International application number: PCT/RU2021/000511
(87) International publication number: WO 2022/164339

(57) **Abstract**

The invention relates to a sampler (1) for connection to a bioreactor (2) filled with a cell culture aqueous solution. The sampler (1) comprises:
• a conduit (10) having:
∘ a first end (101) for connection to a port (21) of the bioreactor (2), said port being intended for collecting samples of the cell culture aqueous solution, and
∘ a second free end (102),

• a hydrophobic air filter (11) for a free egress of air from the conduit (10) to an exterior through the second free end (102), when the conduit (10) is filled with the cell culture aqueous solution.
Said hydrophobic air filter (11) is impermeable to the cell culture aqueous solution.

According to the invention, the sampler (1) comprises a transparent solid container (12) incorporated into the conduit (10) between the first end (101) and the hydrophobic air filter (11). In this case, the transparent solid container (12) is adapted for filling with the cell culture aqueous solution and for a contactless analysis thereof.

## Description

The present invention relates to a field of biotechnology and, in particular, to accessories for equipment used in working with pathogenic microorganisms (infectious agents), for example during a development and manufacturing of immunobiological drugs. Such equipment may be a bioreactor (in disposable or reusable version) filled with a nutrient (for cell culture) medium, such as an aqueous solution. This bioreactor (also called "fermenter") provides a multiplication of pathogenic microorganisms, e.g. viruses, on the culture of cells in the nutrient medium (aqueous solution) under controlled conditions of sterility, stirring intensity, continuous blowing with sterile air, constant temperature, in various volumes: from several hundred milliliters to thousands of liters.

The invention relates to an accessory such as a bioreactor sampling tool. It concerns samples of an aqueous solution with a cell culture contaminated with pathogenic microorganisms. Such instrument is called a sampler. This sampler is adapted in an operational state for connection to a bioreactor filled with a cell culture aqueous solution. The sampler comprises a conduit having:
- a first end adapted for connection to a port of the bioreactor, said port being intended for collecting samples of the cell culture aqueous solution, and
- a second free end opposite the first end.

Also, the sampler comprises a hydrophobic air filter disposed within the conduit between its first end and its second free end. This hydrophobic air filter is adapted for a free egress of air from the conduit to an exterior through the second free end under an effect of a pressure differential between the first end and the second free end, when the conduit of the sampler is filled, in the operational state, with the cell culture aqueous solution from the bioreactor. In this case, the hydrophobic air filter is impermeable to the cell culture aqueous solution.

The sampler described above is known. As mentioned above, operators (scientists, developers, and manufacturers) use both suspension and adhesion cultures cultured using microcarriers during scientific experiments with pathogen-infected cell cultures or during a development and manufacturing of vaccines involving pathogen production. Once infected with a pathogen, it is necessary to monitor cell culture conditions. For example, in the production of viral pathogens that cause a cell lysis, i.e. a cell death, a maximum yield of pathogens can often be harvested after destruction of a vast majority of cells. One of check points during manufacturing of viral preparations is a visual inspection with an optical microscope of the infected cell culture, signaling a correct flow of the process and a need to proceed to a next step of the manufacturing process. For such control, the first end of the known sampler is first connected to a special sampling port, which, as a rule, is available in all bioreactors, regardless of their design features. Next, the operator performs the actual sampling, in which a sample of an aqueous solution of an infected cell culture of a certain volume flows, e.g. by gravity, from the bioreactor into the sampler, gradually pushing air out of the second free end of the piping through a hydrophobic air filter. Then, the operator disconnects the first end from the bioreactor and carefully (i.e., taking care not to spill the contents of the piping around him/her) drains a portion of the collected sample onto a slide for subsequent visual examination using an optical microscope. During such manipulations of the operator with the selected sample, the contents of the bioreactor are in contact with the environment, which increases the risks of infection of the operator and/or release of the pathogen into the working room (including on the measuring equipment, complete and/or regular disinfection of which can be very problematic), as well as further spread of the pathogen outside the working room, which creates a danger to the public. To reduce these risks when working with a known sampler it is necessary to:
- comply with the increased level of operator protection (in particular, constantly check the integrity and functionality of the operator's special protective suit),
- limit the number of operators in the same work area at the same time,
- ensure the cleanliness of the working area and the instruments used for research,
even for routine and low-skilled operator sampling, which is not optimal.

Based on this original observation, the present invention is mainly intended to provide a sampler which at least mitigates at least one of the above-mentioned disadvantages. In order to achieve this aim, the sampler corresponding to the general description given in the introduction above is characterized essentially in that it comprises a transparent solid container cut into the conduit between the first end and the hydrophobic air filter. In these terms, the transparent solid container is adapted:
- to be filled with aqueous solution with cell culture, and
- for non-contact analysis of this aqueous solution with cell culture (using an analyzer).

Due to the transparent solid container, which is an integral part of the conduit, it is possible to carry out the process of viewing and/or instrumental analysis of a sample of cell culture infected with pathogens from the sampler without disconnecting it from the bioreactor, i.e. without contact of the bioreactor contents with the environment, which reduces the risk of pathogen release from the bioreactor into the workroom and/or onto the operator. This contributes to the creation of aseptic conditions when the operator performs sampling with pathogenic biological agents of groups II, III and IV (according to the classification used, for example, in the sanitary and epidemiological rules SR 1.3.2322-08 and SR 1.3.3118-13) in working rooms with the first, second, third biosafety level (in English, "1-3 biosafety level" or, for short, BSL-1, BSL-2, BSL-3).

In addition, the ability to analyze the sample inside a transparent solid container without transferring the sample from the conduit and even without disconnecting the sampler from the bioreactor minimizes the time between sample collection and visual inspection. At the same time, the aqueous solution with pathogen-infected cell cultures does not have time to cool down. In addition, frictional contact of the cells with the piping is minimized. Both of these factors contribute to the accuracy of visual inspection, as cell destruction due to thermal shock and/or mechanical damage due to cell interaction with each other (or with the inner surface of the conduit) is minimized.

The ability to take samples without disconnecting the sampler from the bioreactor, as well as the ability to quickly monitor the sample without allowing it to cool, allows the sample to be returned to the bioreactor immediately after analysis, keeping its temperature and sterility essentially unchanged. This extends the functionality of the sampler.

Advantageously, the transparent solid container is in the form of a quartz cuvette. This quartz cuvette is adapted to be placed in a spectrophotometer.

Due to this advantageous feature, it is possible to monitor various stages of viral drug production using non-contact (i.e. non-invasive) instrumental spectrophotometric measurements. In particular, it is possible to study the presence of cell debris after infection with pathogens. Also, spectrophotometric analysis allows the operator to assess the density and condition of infected suspension cultures and pathogenic microbial cultures. This expands the functionality of the sampler.

Advantageously, the transparent solid container is adapted to be placed on a slide of an optical microscope.

Due to this advantageous feature, the operator can place the transparent solid container on the slide of the optical microscope in advance, i.e. even before sampling. This allows visual inspection to be carried out almost immediately after the aqueous solution with pathogen-infected cell cultures from the bioreactor enters the conduit and, consequently, the transparent solid container. Shortening the time between the moment of sampling and the moment of visual inspection with an optical microscope contributes to the accuracy of visual inspection, because the destruction of cells due to thermal shock and/or due to their mechanical damage in interaction with each other (or with the inner surface of the conduit) is minimized.

Advantageously, the transparent solid container is made of glass.

The use of glass improves the transparency of the solid container and thus facilitates visual observations of the selected aqueous solution with pathogen-infected cell cultures using an optical microscope. In addition, the glass is resistant to sterilization.

As an alternative to glass, the transparent solid container may be made of plastic.

With comparable (in order of magnitude) transparency of glass and plastic, the latter has a lower thermal conductivity, which means that its ability to conduct heat is lower. Accordingly, the use of plastic instead of glass (as a material for a transparent solid container) allows to reduce thermal losses of aqueous solution with cell cultures infected with pathogens at the moment of its getting into a transparent solid container. This contributes to the accuracy of visual control, because the destruction of cells due to thermal shock in a transparent solid container is minimized. In addition, the plastic is resistant to sterilization.

Advantageously, the transparent solid container comprises an analysis zone in the form of a flat slit extending along an axis of the conduit. In this case, an inner thickness of this flat slit is comprised in a first interval from 1 mm to 4 mm: 1 mm ≤ α ≤ 4 mm.

Due to this advantageous feature, the aqueous solution with cell cultures infected with pathogens is distributed inside the transparent solid container as a thin liquid film, the characteristic thickness of which is determined by the inner thickness of the flat slit. For the purposes of optical analysis, the transparent solid container is placed on the slide of the optical microscope so that the axis of the conduit is parallel to the plane of the slide. αIn this case, the light flux of the optical microscope passes through the liquid film filling the flat slit perpendicular to the plane of the slide: having in mind the small (in comparison with the length along the conduit axis) inner thickness of this flat slit, the optical path length and the geometric path length traveled by the light practically coincide. This simplifies the visual analysis of the sample. This selective shape (in the form of a flat slit) of the transparent solid container is particularly adapted for culture control of cells grown on microcarriers (e.g., in the form of optically transparent beads). The small (compared to the length along the conduit axis) inner thickness α of this flat slit limits the number of microcarriers that can be packed on top of each other inside the liquid film perpendicular to the conduit axis, i.e. along the passage of the light flux. This facilitates a more accurate determination with an optical microscope of the state of pathogen-infected cells located on the microcarriers.

The sampler is preferably adapted for disposable use.

The use of a disposable sampler facilitates the operator's routine disposal actions and thus makes the operator's job safer.

Predominantly, the piping is made of flexible material.

This allows the operator to easily move (raise/lower/close/remove) the transparent solid container relative to the bioreactor to facilitate sample collection or discharge back into the bioreactor after control.

Advantageously, the sampler is adapted for sterilizing in an autoclave at a temperature T of at least 120°C during at least 1 hour.

Such sterilization conditions ensure that pathogens are guaranteed to be destroyed on the external and internal surfaces of the sampler (also before disposal of the sampler by, for example, mechanical destruction). Thus, this advantageous feature contributes to increased safety when the operator is working with the sampler. In addition, it allows the sampler to be reused many times, thereby reducing the amount of waste sent for disposal.

Advantageously, the sampler is adapted for steam sterilizing at a temperature T comprised in a second interval from 120°C to 160°C during at least 1 hour.

Such sterilization conditions ensure that pathogens are guaranteed to be destroyed on the external and internal surfaces of the sampler (also before disposal of the sampler by, for example, mechanical destruction). Thus, this advantageous feature contributes to increased safety when the operator is working with the sampler. In addition, it allows the sampler to be reused many times, thereby reducing the amount of waste sent for disposal.

Advantageously, the sampler is adapted for sterilizing with ionizing gamma radiation.

Such sterilization conditions ensure that pathogens are guaranteed to be destroyed on the external and internal surfaces of the sampler (also before disposal of the sampler by, for example, mechanical destruction). Thus, this advantageous feature contributes to increased safety when the operator is working with the sampler. In addition, it allows the sampler to be reused many times, thereby reducing the amount of waste sent for disposal.

Advantageously, the second free end of the conduit is adapted for connection to an air pump. In this case, the sampler in the operational state E₁ is adapted for operating under a pressure P in the conduit comprising in a third interval from 0,1 bar to 2,0 bar: 0,1 bar ≤ P ≤ 2,0 bar.

With an air pump connected to the second free end of the conduit, a controlled discharge can be created in the conduit to draw a sample from the bioreactor or, conversely, a controlled compression can be created to return the sample back to the bioreactor. This simplifies the operator's work.

Advantageously, the transparent solid container has a wall thickness γ less than or equal to 1 millimeter: γ ≤ 1 mm.

The wall thickness γ of a transparent solid container affects its rigidity, transparency, and thermal conductivity. The specified threshold (1 mm) of the maximum wall thickness γ of a transparent solid container is a compromise: exceeding this threshold is not optimal, because it worsens the transparency of the solid container without a significant gain in stiffness and thermal conductivity.

Advantageously, the sampler comprises a sterilizing air filter arranged inside the conduit between the hydrophobic air filter and the second free end of the conduit. Said sterilizing air filter is adapted for sterilizing an external air forced inside the conduit through the second free end by an air pump when the sampler is emptied back into the bioreactor after the contactless analysis of the cell culture aqueous solution.

The presence of such sterilizing air filter makes it possible to exclude the penetration of viruses and bacteria inside the bioreactor from the external air blown into the conduitthrough the second free end when the sampler is emptied back into the bioreactor. This extends the functionality of the sampler.

Advantageously, the sterilizing air filter has a porosity Ω comprising in a fourth interval from 0,1 micrometer to 0,2 micrometer: 0,1 µm ≤ Ω ≤ 0,2 µm.

The applicant has experimentally determined that these selective porosity values Ω from 0,1 micrometer to 0,2 micrometer of the sterilizing air filter provide effective filtration of viruses and bacteria from the outside air of the workroom. This extends the functionality of the sampler.

Advantageously, the sampler comprises a first locking element arranged between the transparent solid container and the first end of the conduit and adapted for locking the conduit from the side of the first end of the conduit.

The first locking element allows to close the conduit from the bioreactor side. This extends the functionality of the sampler, especially in its disposable version, because it allows the operator not to pour the sample back into the bioreactor after its analysis, but - having previously closed the conduit from the bioreactor side using the first locking element - to immediately dispose of the sampler (together with the remaining sample in the conduit). This protocol increases operator safety when working with cell cultures contaminated with highly dangerous pathogens (in particular, viruses from the second, third and fourth pathogenicity groups).

Advantageously, the sampler comprises a second locking element arranged between the hydrophobic air filter and the second end of the conduit and adapted for locking the conduit from the side of the second free end of the conduit.

The presence of the second locking element allows to reliably close the conduit from the side of its second free end, thus eliminating the emergency ingress of the sampled sample into the working room in case of accidental damage to the hydrophobic air filter. This expands the functionality of the sampler and increases operator safety when working with cell cultures contaminated with particularly dangerous pathogens (in particular, viruses from the second, third and fourth pathogenicity groups).

Advantageously, the second locking element is arranged on the side of the second free end of the conduit between the hydrophobic air filter and the sterilizing air filter.

This arrangement of the second locking element allows the conduit to be securely closed at its second free end, in the segment located along the conduit axis after the hydrophobic air filter before the sterilizing air filter. This eliminates contact of the collected sample with the sterilizing air filter in the event of accidental damage to the hydrophobic air filter. This expands the functionality of the sampler and increases operator safety when working with cell cultures contaminated with highly dangerous pathogens (in particular, viruses from the second, third and fourth pathogenicity groups).

Advantageously, the sampler comprises a retainer (for example, in the form of one or more elastic rings) associated with the transparent solid container and adapted to secure the transparent solid container to the analyzer of the cell culture aqueous solution from the bioreactor.

Such a clamp provides secure fixation of the transparent solid container to the analyzer of aqueous solution with cell culture from a bioreactor, for example, to the slide of an optical microscope. This expands the functionality of the sampler and increases operator safety when working with cell cultures contaminated with particularly dangerous pathogens (in particular, viruses from the second, third and fourth pathogenicity groups).

Advantageously, the sampler comprises a first connector arranged at a first end of the conduit and adapted for first quick-release connection of the conduit to a port of the bioreactor for sampling an aqueous solution containing a cell culture.

The first connector allows the sampler to be attached to any standard bioreactor port using the first quick-release connection. This expands the functionality of the sampler, minimizes the risk of operator error when connecting the sampler and bioreactor to each other, and speeds up the routine sampling operation, thereby reducing the total time the operator spends in the work area, which contributes to increased operator safety during routine work.

Advantageously, the sampler comprises a second connector arranged at the second end of the conduit and adapted for a second quick release connection of the conduit to the air pump.

A second connector allows the air pump to be attached to the sampler using a second quick release coupling. This expands the functionality of the sampler, minimizes the risk of operator error when connecting the sampler and air pump to each other, and speeds up the routine sampling operation, thereby reducing the total time the operator spends in the work area, which contributes to increased operator safety during routine work.

In one possible embodiment, the first end of the conduit may be adapted for connection by welding to the port of the bioreactor, said port being intended for collecting samples of the cell culture aqueous solution.

The use of welding allows manufacturers to make the sampler an integral part of the disposable bioreactor. This expands the functionality of the sampler and speeds up the routine sampling operation, thereby reducing the cumulative time the operator spends in the work area, which contributes to increased safety in routine work.

Advantageously, a length L of the conduit between its first end and the transparent solid container does not exceed 10 meters: L ≤ 10 m.

This length L of the conduit (up to and including 10 meters) helps to optimize the operator's movements in the workroom, where the bioreactor and the analysis instruments (optical microscope, spectrometer) can be separated from each other. In addition, the conduit length margin allows the bioreactor to be located in the first working room and the sample measurements to be carried out in the adjacent separate second working room: this contributes to the operator's safety during routine work.

In one possible embodiment of the sampler, the length L of the conduit between its first end and the transparent solid container may be in the range from 0,5 meters to 1,5 meters: 0,5 m ≤ L ≤ 1,5 m.

The length L of the conduit from 0,5 meters to 1,5 meters allows minimizing the sample volume taken by the sampler from the bioreactor. In addition, this length L of the conduit is short enough: the sample of aqueous solution with pathogen-infected cell cultures does not have time to cool down. In addition, frictional contact of cells with the conduit is minimized. Both of these factors contribute to the accuracy of visual inspection, as cell destruction due to thermal shock and/or mechanical damage due to cell interaction with each other (or with the inner surface of the conduit) is minimized.

Predominantly, the conduit has a polished inner surface.

This advantageous feature contributes to minimizing frictional contact of the cells with the conduit, which ultimately contributes to the accuracy of visual inspection, since the destruction of the cells due to their mechanical damage in interaction with the inner surface of the conduit during sampling is minimized.

Predominantly, the conduit has a chemically neutral inner surface.

This contributes to the accuracy of visual inspection, since there is no cell destruction due to chemical attack of the internal surface of the conduit during sampling.

Other distinctive features and advantages of the invention are clearly derived from the description set forth below for illustration and without being restrictive, with reference to the accompanying figures, in which:

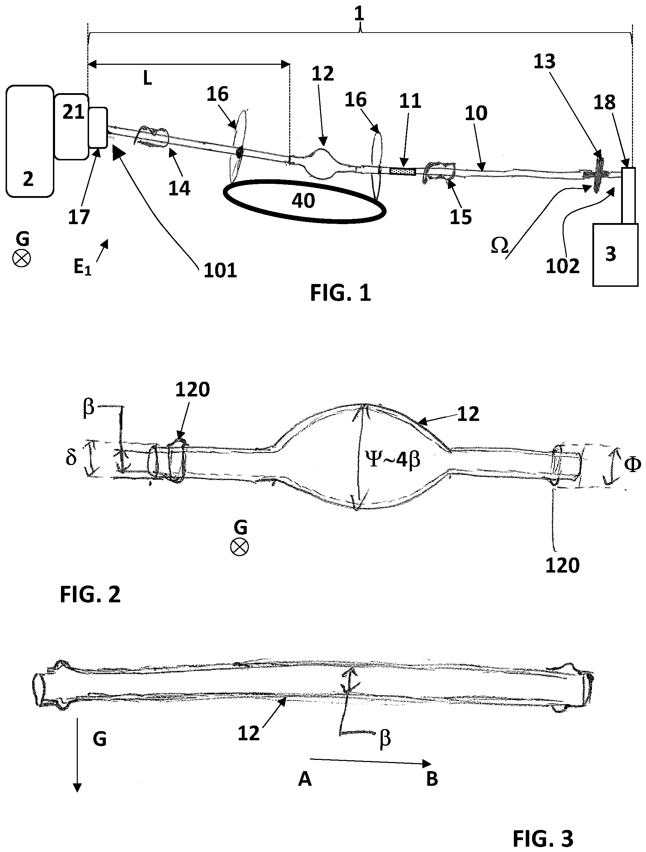

- figure 1 schematically shows in a simplified top view (in a plane perpendicular to the gravity G) a sampler according to the invention in its operational state E₁, i.e. connected to the bioreactor;
- figure 2 schematically depicts in a simplified top view (in a plane perpendicular to the gravity G and coinciding with the plane of the slide of an optical microscope used for visual analysis of samples) a transparent solid container of the sampler, according to a first embodiment thereof;
- Figure 3 schematically depicts in a simplified side view (in a plane coincident with the force of the gravity G and perpendicular to the plane of the slide of the optical microscope used for visual analysis of samples) a transparent solid container of the sampler, according to a first embodiment thereof;

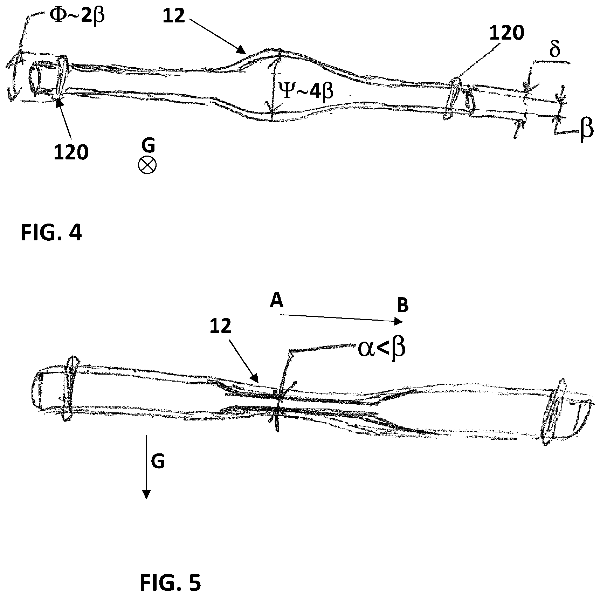

- figure 4 schematically depicts in a simplified top view (in a plane perpendicular to the force of the gravity G and coinciding with the plane of the slide of an optical microscope used for visual analysis of samples) a transparent solid container of the sampler, according to a second embodiment thereof;
- figure 5 schematically depicts in a simplified side view (in a plane coinciding with the force of the gravity G and perpendicular to the plane of the slide of the optical microscope used for visual analysis of samples) a transparent solid container of the sampler, according to the second embodiment, wherein the analysis zone has the form of a flat slit extended along the axis of the conduit.

As mentioned previously and illustrated in figures 1 to 5, the invention relates to a sampler 1 adapted in operational state E₁ for connection to a bioreactor 2. The latter enables a multiplication of pathogenic microorganisms, such as viruses, on a culture of cells contained within the bioreactor 2 in a nutrient medium (aqueous solution) under controlled conditions in terms of sterility, in terms of stirring intensity, in terms of continuous purging with sterile air, and in terms of constant temperature. The bioreactors 2 may be in various designs (disposable, e.g., in the form of a plastic bag, or reusable, e.g., in the form of a glass tank or flask). The bioreactors 2 can be designed in various volumes, ranging from a few hundred milliliters to thousands of liters. Cells can be cultured on suspension cells or on microcarriers, for example in the form of polymer beads.

In a non-operational state (not shown), the sampler 1 is disconnected from the bioreactor 2. It may be in this non-operational state as a separate accessory until it is connected to bioreactor 2 (i.e., until a sample is taken). Alternatively, the sampler 1 may remain in a non-operational state after disconnection from the bioreactor 2 (i.e., after sample collection) pending further sterilization and/or or disposal.

As shown in figure 1, the sampler 1 comprises:
- a conduit 10 having:
   ∘ a first end 101 adapted for connection to a port 21 of the bioreactor 2, said port being intended for collecting samples of the cell culture aqueous solution with cell, and
   ∘ a second free end 102 opposite the first end 101,
- a hydrophobic air filter 11 disposed within the conduit 10 between its first end 101 and its second free end 102.
In this case, the hydrophobic air filter 11 is adapted for a free egress of air from the conduit 10 to an exterior through the second free end 102 under an effect of a pressure differential between the first end 101 and the second free end 102, when the conduit 10 of the sampling device 1 is filled (e.g. by the gravity G), in the operational state E₁, with the cell culture aqueous solution from the bioreactor 2. Under these conditions, the hydrophobic air filter 11 is impermeable to the cell culture aqueous solution: this prevents the sample from spilling out of the second free end to the outside of the working space (not shown).

According to the invention (fig. 1), the sampler 1 comprises a transparent solid container 12 incorporated into the conduit 10 between the first end 101 and the hydrophobic air filter 11. In these circumstances, the transparent solid container 12 is adapted for filling with said cell culture aqueous solution and for a contactless analysis of said cell culture aqueous solution. This analysis is performed by an operator using an analyzer 40 (fig. 1), that is, using one or more instruments that are adapted to measure properties, composition, structure of the sample. Such analyzer 40 may be, for example, a spectrophotometer. In this case, the transparent solid container 12 may be in the form of a quartz cuvette (not shown). This quartz cuvette is adapted to be placed in a cuvette holder of the spectrophotometer (not shown).

Also, the analyzer 40 may be, for example, an optical microscope (not shown). In this case, the transparent solid container 12 may be made of glass or plastic (figs. 2-5). This transparent solid container 12 may be adapted to be placed on a slide (not shown) of the optical microscope.

A first embodiment of the transparent solid container 12 is shown in figs. 2-3. In this first embodiment, the transparent solid container 12 has the shape of a cylinder (fig. 3) extending along the axis AB of the conduit 10, which in the analysis zone comprises a flat (in the plane perpendicular to the gravity force G in fig. 2) elliptically shaped extension. In this case, an inner diameter β of this cylinder (measured, as shown in fig. 3, along the force of gravity G) is comprised within a range from 1 mm to 6 mm : 1 mm ≤ β ≤ 6 mm, and the flat elliptical extension has a characteristic maximum dimension Ψ (measured, as shown in fig. 2, in a plane perpendicular to the force of gravity G) exceeding the inner diameter approximately (in order of magnitude) four times: Ψ ~ 4β. An outer diameter δ of this cylinder (measured, as shown in fig. 3, along the force of gravity G) is comprised within the range from 3 mm to 8 mm: 3 mm ≤ δ ≤ 8 mm, and a thickness γ of the walls of the transparent solid container 12 is determined by the formula γ = (δ - β)/2. In practice, this thickness γ of the walls is less than or equal to 1 millimeter: γ ≤ 1mm.

An advantage of the first embodiment of the transparent solid container 12 is the simplicity and rapidity of its manufacture in mass production: such a shape is easily obtained by glass blowing or thermal molding of corresponding transparent thermoplastic plastics.

As shown in figs. 2-3, opposing ends of the cylinder may include thickenings 120 (e.g., in the form of a ring molded onto the outer surface of the transparent solid container 12) designed to securely fasten the transparent solid container 12 to the conduit 10. In this case, an outer diameter Φ of the thickening 120 (measured, as shown in fig. 2, perpendicular to the force of gravity G) corresponds approximately (in order of magnitude) to two inner diameters of the cylinder: Φ ~ 2β. This increases operator safety when handling the sampler 1.

A second embodiment of the transparent solid container 12 is shown in figs. 4-5. Analogous to the first embodiment described above, in this second embodiment, the transparent solid container 12 has the shape of a cylinder (fig. 5) extending along the axis AB of the conduit 10, which in the analysis zone contains a flat (in the plane perpendicular to the force of gravity G in fig.4) elliptically shaped extension. In this case, the inner diameter β of this cylinder (measured as shown perpendicular to gravity G in FIG. 4) is comprised within the range from 1 mm to 6 mm: 1 mm ≤ β ≤ 6 mm, and the elliptically shaped flat extension has a characteristic maximum dimension Ψ (measured as shown in fig. 4 in the plane perpendicular to the gravity G) exceeding the inner diameter β by a factor of about (in order of magnitude) four: Ψ ~ 4β. The outer diameter δ of this cylinder (measured, as shown in fig. 3, along the force of gravity G) is comprised within the range of 3 mm to 8 mm : 3 mm ≤ δ ≤ 8 mm, and the thickness γ of the walls of the transparent solid container 12 is determined by the formula γ= (δ - β)/2. In practice, this wall thickness γ is less than or equal to 1 millimeter: γ ≤ 1mm. As shown in fig. 5, in this second embodiment, the analysis zone which contains, as indicated above, a flat (in a plane perpendicular to gravity G in fig. 4) elliptically shaped extension (fig. 4), is additionally a flat slot extending along the axis AB of the conduit 10. In this case, the inner thickness α of this flat slot is smaller than the inner diameter of the cylinder (i.e. α < β) and is predominantly in the first range of one to four millimeters: 1 mm ≤ α <_ 4 mm.

Advantageously, the sampler 1 is adapted for disposable use.

The conduit 10 may be made of a flexible material.

The sampling device 1 can be adapted:
- for sterilizing in an autoclave at a temperature T of at least 120°C during at least 1 hour, and/or
- for steam sterilizing at a temperature T comprised in a second range from 120°C to 160°C (120°C ≤ T ≤ 160°C) during at least 1 hour, and/or
- for sterilizing with ionizing gamma radiation.

As shown in FIG. 1, the second free end 102 may be adapted to be connected to an air pump 3 (e.g. in the form of a medical syringe of appropriate volume). In this case, the sampler 1 in the operational state E₁ is adapted to operate at a pressure P in the conduit 10 comprising in a third interval from 0,1 bar and 2,0 bar: 0,1 bar ≤ P ≤2,0 bar.

As shown in fig. 1, the sampler 1 may comprise a sterilizing air filter 13 positioned within the conduit 10 between the hydrophobic air filter 11 and the second free end 102 of the conduit 10. This sterilizing air filter 13 is adapted to sterilize external air forced inside the conduit 10 through the second free end 102 by the air pump 3 when the sampler 1 is emptied back into the bioreactor 2 after non-contact analysis of the aqueous solution containing the cell culture.

Advantageously, the sterilizing air filter 13 has a porosity Ω, comprising in a fourth interval from 0,1 micrometer to 0,2 micrometer: 0,1 micrometer ≤ Ω ≤ 0,2 micrometer.

As shown in FIG. 1, the sampling device 1 may comprise a first locking element 14 disposed between the transparent solid container 12 and the first end 101 of the conduit 10 and adapted to lock the conduit 10 at a side of the first end 101 of the conduit 10.

As shown in fig. 1, the sampling device 1 may include a second locking element 15 disposed between the hydrophobic air filter 11 and the second end of the conduit 102 and adapted to lock the conduit 10 to the side of the second end 102 of the conduit 10.

Advantageously, the second locking element 15 is arranged on the side of the second free end 102 of the conduit 10 between the hydrophobic air filter 11 and the sterilizing air filter 13.

As shown in fig. 1, the sampler 1 may include a retainer 16 associated with the transparent solid container 12 and adapted to secure the transparent solid container 12 to the analyzer 40 of an aqueous solution containing cell culture from the bioreactor 2.

As shown in fig. 1, the sampler 1 may include a first connector 17 disposed at a first end 101 of the conduit 10 and adapted for first quick-release connection of the conduit 10 to a port 21 of the bioreactor 2 for sampling an aqueous solution containing a cell culture.

As shown in fig. 1, the sampler 1 may include a second connector 18 disposed at the second end 102 of the conduit 10 and adapted for a second quick-release connection of the conduit 10 to the air pump 3.

The first end 101 of the conduit 10 may be adapted to be connected by heat welding (not shown) to a port 21 of the bioreactor 2 for sampling an aqueous solution with cell culture.

Advantageously, the length L of the conduit 10 between its first end 101 and the transparent solid container 12 does not exceed 10 meters: L ≤ 10 m.

Advantageously, the length L of the conduit 10 between its first end 101 and the transparent solid container 12 is comprised in a spot range from 0,5 meters to 1,5 meters: 0,5 m ≤ L ≤ 1,5 m.

Advantageously, the conduit 10 is a polished and chemically neutral inner surface.

Two examples of the operation of the sampler 1 according to the invention are given below.

### Example 1.

The operator takes the sampler 1 in a disposable and inoperable state from the accessory box for the bioreactor 2. This sampler 1 comprises a conduit 10 (in the form of a flexible hose with a polished and chemically neutral inner surface), a transparent solid container 12, a hydrophobic air filter 11, and a sterilizing air filter 13. The operator locks the conduit 10 with a first locking element 14 on the side of the first end 101, and with a second locking element 15 on the side of the second free end 102. Next, the operator brings the sampler 1 into the operational state E₁, shown in fig. 1, by connecting the first end 101 of the conduit 10 to the bioreactor 2 using the first connector 17 and the second end 102 of the conduit 10 to the air pump 3 (in the form of an empty sterile syringe of 50 mL to 100 mL volume) using the second connector 18. Then, the operator creates, by activating the air pump 3 (i.e., by applying hand pressure to the syringe piston), a predetermined vacuum in the conduit 10. After that, the operator unclamps the first locking element 14 and the second locking element 15: under the action of the predetermined vacuum, the aqueous solution with the cell culture rushes from the bioreactor 2 into the conduit 10 and further into a transparent solid container 12 in the form of a quartz cuvette (not shown). After filling this cuvette with sample, the operator places the cuvette in the cuvette holder (otherwise known as the "nest") of the spectrophotometer. Next, the operator performs a spectrophotometric analysis of the contents of the bioreactor 2. After the end of the analysis by activating the air pump 3 (i.e. by reverse stroke of the syringe piston) the cell suspension with pathogen is brought from the sampler 1 back into the bioreactor 2. Next, the operator locks the conduit 10 with the first locking element 14 at the side of the first end 101. Thereafter, the operator places the sampler 1 in an inoperable state by disconnecting the first end 101 of the conduit 10 from the bioreactor 2 using the first connector 17. Finally, the disposable sampler 1 together with the air pump 3 connected thereto is disposed.

### Example 2.

The operator takes a reusable and inoperable sampler 1 from the bioreactor accessory box 2. This sampler 1 comprises a conduit 10 (in the form of a flexible hose with a polished and chemically neutral inner surface), a transparent solid container 12, a hydrophobic air filter 11, and a sterilizing air filter 13. The operator locks the conduit 10 with a first locking element 14 on the side of the first end 101, and with a second locking element 15 on the side of the second free end 102. ₁Next, the operator brings the sampler 1 into the operating state E , shown in FIG. 1, by connecting the first end 101 of the conduit 10 to the bioreactor 2 using the first connector 17 and the second end 102 of the conduit 10 to the air pump 3 (in the form of an empty sterile syringe of 50 mL to 100 mL volume) using the second connector 18. Then, the operator creates, by activating the air pump 3 (i.e., by applying hand pressure to the syringe piston), a predetermined vacuum in the conduit 10. After that, the operator unclamps the first locking element 14 and the second locking element 15: under the action of the predetermined discharge of aqueous solution with cell culture rushes from the bioreactor 2 into the conduit 10 and then into a transparent solid container 12 in the form of a cylinder (Fig. 5), extended along the axis AB of the conduit 10, which in the analysis zone contains a flat (in the plane perpendicular to the force of gravity G in fig. 4) elliptical expansion (fig. 4). This elliptically shaped extension holds a narrow flat slot extending along the axis AB of the conduit 10, as shown in fig. 5.

The operator fills 1/3 of the entire volume of the transparent solid container 12 with the sample. Next, the operator re-locks the conduit 10 with a first locking element 14 on the side of the first end 101, and with a second locking element 15 on the side of the second free end 102. Then, the operator secures the one-third filled transparent solid container 12 to the optical microscope slide by means of a retainer 16 (in this example, by means of two rubber bands located on either side of the transparent solid container 12 along the axis AB of the conduit 10). The operator then visually analyzes the state of the cell culture within the transparent solid container 12 using the optical microscope.

After the analysis is completed, the operator unclamps the first locking element 14 and the second locking element 15, and then by activating the air pump 3 (i.e. by reversing the stroke of the syringe piston) the cell suspension with the pathogen is introduced from the sampler 1 back into the bioreactor 2. Next, the operator re-locks the conduit 10 with a first locking element 14 on the side of the first end 101, and with a second locking element 15 on the side of the second free end 102. Thereafter, the operator places the sampler 1 in an inoperable state by disconnecting the first end 101 of the conduit 10 from the bioreactor 2 using the first connector 17 and the second end 102 of the conduit 10 from the air pump 3 using the second connector 18. Finally, the reusable sampler 1 is sterilized by ionizing gamma radiation for further use.

The main advantages of the sampler 1 according to the invention are summarized below:
- minimizes the risk of release of infectious agent into the environment and, in particular, on the operator, on measuring instruments, in the working room, because during any manipulations with the sample (taking from the bioreactor 2/analysis of the sample/return of the sample to the bioreactor 2) the cell culture studied by the operator does not come into contact with the external environment. Thus, the risk of contamination is reduced and, consequently, the working conditions and safety of all working personnel operating the bioreactor 2 and, in particular, the operator who performs routine operations of sample collection, analysis and subsequent return to the bioreactor 2 are improved;
- the risk of contamination of the bioreactor 2 itself is minimized when the sample is returned to the bioreactor 2 after it has been analyzed by an operator using an analyzer (e.g., an optical microscope with a magnification of x5, x10, x20, and/or a spectrophotometer), because even during the analysis of the sample there is no contact of the contaminated cell culture (i.e., infectious agent) with the environment;
- minimizes the consumption of supplies for daily monitoring of infected cells, reduces waste in routine work to monitor the multiplication of pathogens, such as viruses, on cell culture media (aqueous solution) under controlled conditions in bioreactor 2.

## Claims

1. A sampler (1) adapted in an operational state (E₁) for connection to a bioreactor (2) filled with a cell culture aqueous solution, the sampler (1) comprising:
• a conduit (10) having:
∘ a first end (101) adapted for connection to a port (21) of the bioreactor (2), said port being intended for collecting samples of the cell culture aqueous solution, and
∘ a second free end (102) opposite the first end (101),
• a hydrophobic air filter (11) disposed within the conduit (10) between its first end (101) and its second free end (102),
the hydrophobic air filter (11) being adapted for a free egress of air from the conduit (10) to an exterior through the second free end (102) under an effect of a pressure differential between the first end (101) and the second free end (102), when the conduit (10) of the sampler (1) is filled, in the operational state (E₁), with the cell culture aqueous solution from the bioreactor (2),
the hydrophobic air filter (11) being impermeable to the cell culture aqueous solution,
**characterized in that** the sampler (1) comprises a transparent solid container (12) incorporated into the conduit (10) between the first end (101) and the hydrophobic air filter (11), and **in that** the transparent solid container (12) is adapted for filling with said cell culture aqueous solution and for a contactless analysis of said cell culture aqueous solution with the help of an analyzer (40).

2. The sampler (1) according to claim 1, **characterized in that** the transparent solid container (12) is in the form of a quartz cuvette, and **in that** said quartz cuvette is adapted to be placed in a spectrophotometer.

3. The sampler (1) according to claim 1 or 2, **characterized in that** the transparent solid container (12) is adapted to be placed on a slide of an optical microscope.

4. The sampler (1) according to any one of claims 1 to 3, **characterized in that** the transparent solid container (12) comprises an analysis zone in the form of a flat slit extending along an axis (AB) of the conduit (10), and **in that** an inner thickness (α) of this flat slit is comprised in a first interval from 1 mm to 4 mm.

5. The sampler (1) according to any one of claims 1 to 4, **characterized in that** it is adapted for disposable use.

6. The sampler (1) according to any one of claims 1 to 5, **characterized in that** it is adapted for sterilizing in an autoclave at a temperature (T) of at least 120°C during at least 1 hour.

7. The sampler (1) according to any one of claims 1 to 6, **characterized in that** it is adapted for steam sterilizing at a temperature (T) comprised in a second interval from 120°C to 160°C during at least 1 hour.

8. The sampler (1) according to any one of claims 1 to 7, **characterized in that** it is adapted for sterilizing with ionizing gamma radiation.

9. The sampler (1) according to any one of claims 1 to 8, **characterized in that** the second free end (102) is adapted for connection to an air pump (3), and **in that** the sampler (1) in the operational state (E₁) is adapted for operating under a pressure (P) in the conduit (10) comprising in a third interval from 0,1 bar to 2,0 bar.

10. The sampler (1) according to any one of claims 1 to 9, **characterized in that** it comprises a sterilizing air filter (13) arranged inside the conduit (10) between the hydrophobic air filter (11) and the second free end (102) of the conduit (10), and **in that** said sterilizing air filter (13) is adapted for sterilizing an external air forced inside the conduit (10) through the second free end (102) by the air pump (3) when the sampler (1) is emptied back into the bioreactor (2) after the contactless analysis of the cell culture aqueous solution.

11. The sampler (1) according to claim 10, **characterized in that** the sterilizing air filter (13) has a porosity (Ω) comprising in a fourth interval from 0,1 micrometer to 0,2 micrometer.

12. The sampler (1) according to any one of claims 1 to 11, **characterized in that** it comprises a first locking element (14) arranged between the transparent solid container (12) and the first end (101) of the conduit (10) and adapted for locking the conduit (10) from the side of the first end (101) of the conduit (10).

13. The sampler (1) according to any one of claims 1 to 12, **characterized in that** it comprises a second locking element (15) arranged between the hydrophobic air filter (11) and the second free end of the conduit (102) and adapted for locking the conduit (10) from the side of the second free end (102) of the conduit (10).

14. The sampler (1) according to any one of claims 1 to 13, **characterized in that** it comprises a retainer (16) associated with the transparent solid container (12) and adapted to secure the transparent solid container (12) to the analyzer (40) of the cell culture aqueous solution from the bioreactor (2).

15. The sampler (1) according to any one of claims 1 to 14, **characterized in that** the first end (101) of the conduit (10) is adapted for connection by welding to the port (21) of the bioreactor (2), said port being intended for collecting samples of the cell culture aqueous solution.
